# EUROPEAN PATENT APPLICATION

(11) **EP 1 731 146 A1**
(43) Date of publication of application: **13.12.2006**
(21) Application number: 04717741.5
(22) Date of filing: 05.03.2004
(51) Int. Cl.: A61K 31/198, A61K 31/375, A61P 43/00

(54) **PREVENTION AND MEASURE FOR MITOCHONDRIAL DISEASE**

(71) Applicant: Morishige, Fumie, Sanbu-gun, Chiba 299-3236 (JP)
(72) Inventor: MORISHIGE, Fukumi, Sanbu-gun, Chiba 2993236 (JP)
(74) Representative: Brady, Paul Andrew
(86) International application number: PCT/JP2004/002787
(87) International publication number: WO 2005/084660

(57) **Abstract**

The present invention relates to an agent containing L-arginine, 0.2 to 20 parts by weight of L-ascorbic acid per 1 part by weight of L-arginine, and, if desired, at least one selected from the group consisting of ribonucleic acids, ribonucleotides and ribonucleosides. The agent can treat mitochondrial disease which shows a variety of symptoms caused by dysfunction of mitochondria in cells. L-arginine contained in the agent for treating mitochondrial disease of the present invention increases an NO radical level to thereby dilate the arteries. L-ascorbic acid serves to mitigate a harsh taste and acrid feeling accompanied with the intake of L-arginine and eliminate excessive NO radicals.

## Description

### Technical Field

The present invention provides an agent efficacious for prevention of and countermeasures against diseases collectively called as "mitochondrial disease". The mitochondrial disease shows various symptoms caused by dysfunction of mitochondria in cells.

### Background Art

Mitochondria are organelles present in cells and deeply involved in energy production. Abnormality in heredity of enzymes of the energy production system decreases the functions of mitochondria to induce diseases of various types in, for example, organs such as central nerve, skeletal muscles, heart, eyes, liver, kidneys, large intestine (colon), small intestine, internal ear and pancreata; as well as blood, skin and endocrine glands. These diseases are collectively called as "mitochondrial disease". Such a disease in the brain and muscles is sometimes calls as "mitochondrial encephalomyopathy", because the brain and muscles consume a large quantity of energy, and the dysfunction of mitochondria significantly affects these organs.

The mitochondrial disease is classified in various ways by biochemical abnormalities, clinical symptoms or types of DNA abnormalities. Types named as KSS (chronic progressive external ophthalmoplegia), MERRF (myoclonus epilepsy associated with ragged-red fibers; Fukuhara syndrome), MELAS, Leber's disease, Leigh encephalopathia and Pearson's disease are widely known. Among them, MELAS is a type mainly showing stroke-like episodes, occupies 30% or more of the whole and is believed to be the most frequent type in the mitochondrial disease.

MELAS is a type of the mitochondrial disease which develops in one's childhood with ictal headache (migraine), emesis and/or spasticity in one side of the body and was first clinically reported in Columbia University in 1984. Eighty percent (80%) of subjects with MELAS are reported to have A3243G mutation in tRNALeu(UUR) gene of mitochondrial DNA. This type features stroke-like episodes before one's twenties. The stroke may occur continuously. Some symptoms observed in stroke or attack are transitory, but they may become prolonged unless an appropriate treatment is carried out. One stroke may induce death in some cases.

The mitochondrial diseases can be diagnosed by investigating whether or not mitochondria are abnormal in their shape, function and DNA.

The shape of mitochondria is investigated by sectioning and staining the skeletal muscle and observing the stained sections on an optical microscope. The stained normal mitochondria are indistinctive in cells. The myocyte of patients with the mitochondrial disease shows stained mitochondria as spots inside thereof or as accumulation on the periphery thereof. Such an abnormal myocyte (muscle fiber) is called as ragged-red-fiber: RRF) and is an indicator of whether or not the target is mitochondrial disease.

The function of mitochondria is investigated, for example, by assaying the activity of Complex IV: cytochrome c oxidase (COX), an enzyme of the electron transfer system in mitochondria. Some patients with the mitochondrial disease show dispersed muscular cells having no COX activity in the assay of COX activity on frozen sections of the muscular tissue. This "partial deficiency of COX" is an important indicator indicating mitochondrial abnormality.

In addition, whether or not one suffers from the mitochondrial disease can also be determined by investigating the abnormality of mitochondrial DNA. The studies on mitochondrial DNA have been significantly advanced, and the results thereof are now clinically applied.

Conventional treatments of the mitochondrial disease are roughly classified as two groups, a palliative treatment and a causal treatment.

The palliative treatment is a treatment which comprises treating symptoms caused by the mitochondrial disease by an agent which is known to be efficacious to similar symptoms in diseases other than the mitochondrial disease, such as diabetes and epilepsy. This treatment uses such an agent that has been verified to be efficacious and is sufficiently promising in its efficacy. It does, however, not improve the dysfunction of mitochondria and is not a fundamental cure.

The causal treatment is a treatment in order to improve the dysfunction of mitochondria itself by administrating an agent for activating mitochondria. Such an agent is selected from the viewpoint of activating energy metabolism in mitochondria and includes, for example, dichloroacetic acid (DCA), Neuquinon and vitamin B1.

Dichloroacetic acid acts to increase the activity of pyruvate dehydrogenase (PDHC) and to increase energy metabolism in mitochondria. This agent particularly markedly acts to improve acidosis (acidemia) due to an increased blood lactic acid level. DCA must be coadministered with vitamin B1, because DCA acts to increase the activity of PDHC but to consume vitamin B1. Some reports mention that the administration of DCA inhibits the seizure of the mitochondrial disease and improves the general condition, but others mention that impaired consciousness and liver function disorder occur in a high dose of DCA. Dichloroacetic acid must therefore be administered while always observing the blood level.

Neuquinon is an agent known in the treatment of congestive heart failure and has been reported to be efficacious for the mitochondrial disease in a high dose. Some reports, however, mention that Neuquinon is inefficacious for the mitochondrial disease.

Vitamin B1 has been reported to be remarkably efficacious for part of patients with Leigh encephalopathia, a type of the mitochondrial disease. Some of patients with Leigh encephalopathia show deficiency of vitamin B1-dependent pyruvate dehydrogenase complex, and the administration of vitamin B1 shows remarkable effects in these cases. Vitamin B1 plays an important role on coenzymes in the mitochondrial metabolism and is therefore often administered to patients with the mitochondrial disease without the deficiency of vitamin B1, even when no significant effect is observed.

As is described above, no agent is known to be efficacious for the mitochondrial disease of all the types without showing severe adverse drug actions in the causal treatment of mitochondrial disease. Accordingly, an object of the present invention is to provide an agent which is efficacious for the prevention and treatment of the mitochondrial disease of all the types by activating mitochondria.

### Disclosure of Invention

After intensive investigations, the present inventors have found that coadministration of arginine with vitamin C can activate mitochondria and inhibit adverse effects caused by the administration of arginine and can constitute an agent efficacious for causal treatment of the mitochondrial disease. They have also found that incorporation of ribonucleic acids in addition to arginine and vitamin C enhances the effects on the mitochondrial disease.

Accordingly, the present invention relates to:
- an agent for treating mitochondrial disease, comprising L-arginine and 0.2 to 20 parts by weight of L-ascorbic acid per 1 part by weight of L-arginine, and
- an agent for treating mitochondrial disease, comprising L-arginine, at least one selected from the group consisting of ribonucleic acids, ribonucleotides and ribonucleosides, and 0.2 to 20 parts by weight of L-ascorbic acid per 1 part by weight of L-arginine.

### Best Mode for Carrying Out the Invention

The agent for treating mitochondrial disease of the present invention includes, as a first embodiment, L-arginine and 0.2 to 20 parts by weight of L-ascorbic acid per 1 part by weight of L-arginine.

L-arginine has a chemical formula of C₆H₁₄N₄O₂, a molecular weight of 374.20 and a melting point (decomposition point) of 244°C, is one of basic amino acids and is abundant in basic proteins such as protamine and histone.

L-arginine has been reported to be a source of NO radical which relates to a wide variety of vital functions such as control of blood pressure and prevention of infection. L-arginine has also been found to be involved in various physiologic functions. For example, it is involved in enhancement of detoxication of ammonia in the urea cycle and secretion of endocrine hormones, works as a synthetic material for creatine phosphates, polyamines and proline and as a source of NO radical.

Patients with MELAS, a type of the mitochondrial disease, have been found to have a markedly decreased effective concentration of L-arginine in the acute phase of cerebral stroke. The NO radical level indicating the total amount of NO production significantly decreases with a decreasing level of L-arginine in patients with MELAS. Thus, it can be said that the arteries of the patients with MELAS are hardly dilated. The administration of L-arginine to a patient with the mitochondrial disease to thereby increase NO radicals is expected to be efficacious for treating the mitochondrial disease.

L-arginine, however, has a very harsh taste (acrid, pungent taste that irritates the throat) and is very difficult to take in. The taken L-arginine invites acrid feeling in the stomach (heartburn, vomiturition, nausea or vomiting) and is very uncomfortable. Among such L-arginine compounds, salts of L-arginine with inorganic acids, such as hydrochlorides, nitrates or sulfates, have a very strong harsh taste and invite strong acrid feeling after intake.

In-vivo NO increases after long-term intake of L-arginine, is converted into nitrous acid in the stomach and reacts with a secondary amine contained in food in the stomach to form a nitrosamine compound having mutagenicity (carcinogenicity). An active oxygen species such as superoxide radical anion is often formed in injured cells, and the superoxide radical anion react with the NO radical to form a highly toxic peroxynitrite ion (O=NOO-).

The present inventors, however, have found that these problems associated with the intake of L-arginine can be solved by intake of L-arginine together with L-ascorbic acid and that L-ascorbic acid can enhance the treatment of the mitochondrial disease. The present invention has been accomplished based on these findings.

L-ascorbic acid (vitamin C) is a water-soluble vitamin and is involved in in-vivo redox reactions. Certain animals such as humans and monkeys or apes do not have an enzyme relating to the reaction of an intermediate of L-ascorbic acid. A condition which is called typically as hypoascorbicacidemia or induced oligascorbicacidosis with a decreased level of L-ascorbic acid in the blood generally induces disorder in the immune system, disorder in the blood coagulation system, incontinence to stress due to hypofunction of the adrenal gland, disorder in collagen production, malfunction in the nervous system, or onset of cancer caused by hypofunction of the immune or decreased detoxication capability against carcinogen.

L-ascorbic acid mixed with L-arginine can mitigate the harsh taste of L-arginine and the acrid feeling in the stomach (heartburn, vomiturition, nausea or vomiting) after intake of arginine.

L-ascorbic acid always undergoes autoxidation to release radicals. Excessive NO radicals formed by the administration of L-arginine can be scavenged by the radicals derived from L-ascorbic acid. This prevent the excessive NO radicals formed by the taken L-arginine to convert into harmful nitrosamine compounds or peroxynitrite ions.

At least 0.2 part by weight of L-ascorbic acid must be mixed with 1 part by weight of L-arginine in order to mitigate the harsh taste of L-arginine. If the part by weight of L-ascorbic acid to be mixed is less than 0.2, the harsh taste of L-arginine increases. If the part by weight of L-ascorbic acid exceeds 0.25, the sourness of the mixture increases with an increasing part by weight of mixed L-ascorbic acid. The same tendency is present in the case where L-ascorbic acid is added in order to mitigate the acrid feeling after intake of L-arginine. The acrid feeling can be mitigated by mixing at least 0.2 part by weight of L-ascorbic acid with 1 part by weight of L-arginine.

The mixture at an amount of L-ascorbic acid exceeding 0.25 part by weight per 1 part by weight of L-arginine shows sourness in the agent for treating mitochondrial disease. The sourness is derived from ascorbic acid and is not intolerable for general subjects. This can be understood by the fact that powder of ascorbic acid is commercially available as a nutritional supplementary food that can be taken in routinely. The sourness is considerably reduced by formulating into tablets. Accordingly, if the amount of L-ascorbic acid exceeds the minimal necessary amount thereof to mitigate the harsh taste and acrid feeling of L-arginine, it produces no problem in the present invention. L-ascorbic acid, for example, can be mixed with L-arginine in an amount of 20 parts by weight per 1 part by weight of L-arginine.

Part of L-ascorbic acid to be mixed can be replaced with vitamin C other than L-ascorbic acid, or a derivative and analogue having the same action in vivo as L-ascorbic acid.

The agent for treating mitochondrial disease of the present invention, as a second embodiment, comprises L-arginine, at least one selected from the group consisting of ribonucleic acids, ribonucleotides and ribonucleosides, and 0.2 to 20 parts by weight of L-ascorbic acid per 1 part by weight of L-arginine.

Such ribonucleic acids are known as a nutritional supplementary food and are used for improving symptoms of cerebrospinal diseases. Typical examples of the cerebrospinal diseases are involutional cerebrospinal diseases such as dementia including senile dementia, epilepsy, convulsive attack, encephalopathy, cranial nerve disease, basal ganglia disease, degenerative disease of cerebelli, degenerative disease of spinal cord and myopathy. The administration of ribonucleic acid in addition to L-arginine and L-ascorbic acid works to resolve the disorders caused by the mitochondrial disease particularly in the central nerve system including brain.

In a high dose of ribonucleic acids, the coadministration of ascorbic acid has a tendency to decrease the uric acid level in the blood, as compared with the case where ascorbic acid is not coadministered. This can avoid the symptoms of gout which may be caused by an increased uric acid level in the blood in a patient who has a genetic predisposition to increase the uric acid level by the administration typically of RNA. This is because ascorbic acid decreases the activity of an enzyme relating to the metabolism from hypoxanthine to xanthine to thereby reduce the amount of formed uric acid in the purine excretion system which yields uric acid as a final product. The ascorbic acid added in order to eliminate the harsh taste of arginine also acts to decrease the uric acid level in the blood due to, for example, the intake of RNA to thereby avoid onset of gout.

L-arginine, L-ascorbic acid and mixing ratio thereof are as mentioned above.

L-arginine, L-ascorbic acid and ribonucleic acids for use in the present invention are preferably in the form of a solid suitable for mixing, such as powder or fine powder, crystal or fine crystal, or block that can be easily crushed into fine powder or fine crystal. As L-ascorbic acid, a finely divided powder (e.g., "Vitamin Granule-97", a product of BASF Takeda Vitamin Ltd.) prepared by fluidized bed granulation while adding a small amount, e.g., 3% of an adhesive such as corn starch can be used.

The progress of browning can further be inhibited by adding a powder of a polyol such as xylitol or sorbitol to the mixture.

The mixing is generally carried out by fully pulverizing powders, fine powders, crystals or fine crystals of the individual ingredients so as to yield a homogenous mixture. The mixing is generally associated with pulverization.

The mixing is carried out in an atmosphere of low humidity of 40% or less to minimize oxidation of L-ascorbic acid in an atmosphere containing moisture. A container for the mixing preferably has an inner surface made of a material that is inert to the ingredients to be mixed and is hard, such as earthenwares and other ceramics. An example of the container is a mortar, mill or tube (circular cylinder or prism) which is made of a ceramic such as earthenware, has a smooth or rough inner surface and is durable. A mill-type mixer in which the ingredients is shredded with an iron cutter blade is undesirable, because L-ascorbic acid easily reacts with iron.

The mixing is sufficiently carried out at ordinary temperature, preferably 10°C to 15°C, so as to mix the individual ingredients uniformly. A sufficient time to mix the individual ingredients uniformly is enough as the mixing time. The particle size of the resulting mixture is, for example, 50-mesh, preferably 100-mesh and more preferably 200-mesh sieve pass.

L-ascorbic acid may be coated before mixing with L-arginine. The coated L-ascorbic acid is not in direct contact with L-arginine and does not invite the Maillard reaction. L-ascorbic acid fully coated with an oil or fat can be used as the coated L-ascorbic acid.

Examples of the ribonucleic acids are those derived from brewery yeast extract.

The agent for treating mitochondrial disease of the present invention can be a liquid suitable for drip infusion or injection. The liquid agent for treating mitochondrial disease is efficacious when the subject is unconscious in the acute phase of the mitochondrial disease or is an elderly and cannot take the agent for treating mitochondrial disease orally. Upon such a formulation of a liquid pharmaceutical preparation, the Maillard reaction between an amino acid and a saccharide must be avoided. The Maillard reaction once occurred inhibits the effects of the individual components and deteriorates the taste of the preparation. The present inventors, however, have found that a mixture of L-arginine and L-ascorbic acid as an aqueous solution does not invite the Maillard reaction over several months, if it does not contain cystine and cysteine. They also have found that the duration until the Maillard reaction occurs can be prolonged by adding a sugar alcohol such as xylitol or sorbitol thereto.

The present invention therefore also relates to an agent for treating mitochondrial disease, comprising L-arginine and 0.2 to 20 parts by weight of L-ascorbic acid per 1 part by weight of L-arginine and containing no cysteine, L-arginine and L-ascorbic acid being dissolved in a pharmaceutically acceptable solution.

The agent for treating mitochondrial disease of the present invention serves to increase the activity of mitochondria themselves and can be used in the treatment of the mitochondrial disease of a variety of types as follows, regardless of the types:
chronic progressive external ophthalmoplegia (KSS), MERRF (myoclonus epilepsy associated with ragged-red fibers; Fukuhara syndrome), MELAS, Leber's disease, Leigh encephalopathia, diabetes associated with mitochondrial abnormality and Pearson's disease.

The mitochondrial disease is symptomatologically classified as follows:
1. Nerve system: stroke, cerebellar ataxia, mental retardation, migraine, cortical blindness, spasticity (epilepsy), myoclonus, peripheral neuropathy, dementia
2. Skeletal muscle: ophthalmoplegia externa, muscle weakness, blepharoptosis, easy fatigability
3. Endocrine: diabetes, short stature, retardation in development of secondary sex characters, hypoparathyroidism
4. Cardiovascular system: hypertrophic cardiomyopathy, congestive cardiomyopathy, cardiac conduction block
5. Renal tubular disorder: renal uriniferous tubular acidosis, de Toni-Fanconi syndrome
6. Blood: sideroblastic anemia (Pearson syndrome)
7. Hepatobiliary system: hypertransaminase, hepatic failure, pancreatic achylia
8. Digestive organ: ileus, chronic diarrhea, iterative emesis
9. Visual organ: retinal pigment degeneration, atrophy of optic nerve
10. Auditory organ: perceptive deafness
11. Psychiatry: schizophrenia, behavior disorder, autism

The mitochondrial disease is classified by biochemical abnormalities as follows:
carnitine palmitoyltransferase deficiency, carnitine deficiency, pyruvate carboxylase deficiency, pyruvate dehydrogenase deficiency, fumarase deficiency, α-ketoglutarate dehydrogenase deficiency, Luft's disease, Complex 1 deficiency, Complex 2 deficiency, Complex 3 deficiency, Complex 4 deficiency, Complex 5 deficiency, deficiency of plural complexes.

The mitochondrial disease is classified by clinical symptoms as follows:
chronic progressive external ophthalmoplegia (CPEO) (including Kearns-Sayre syndrome), mitochondrial disease associated with myoclonus, mitochondrial disease associated with stroke-like episodes, Leber's disease, Leigh encephalopathia, Pearson disease, NARP.

Classification by DNA abnormalities:
abnormality of nuclear DNA, abnormality of mtDNA, (defect-duplication)-(point mutation)-(deficiency).

The dose of the agent for treating mitochondrial disease of the present invention can be appropriately selected, for example, by observing the NOₓ level in the urine using a tes-tape. More specifically, if the symptom of the mitochondrial disease is not improved at a certain does, the dose may be increased within a range in which the NOₓ level does not change. In contrast, the dose may be decreased when the NOₓ level increases. The dose of the agent for treating mitochondrial disease may be as high as 30 g in terms of L-arginine in some cases. In this connection, the blood NOₓ level does not work as an indicator of the dose, because arginine is rapidly pooled in the cerebral nerve system and muscle.

The present invention will be illustrated in further detail with reference to several examples below which should never be construed to limit the scope of the present invention.

### PREPARATION EXAMPLE 1

In a ceramic mortar were placed and sufficiently ground 5 g of powdery L-arginine and 1 g of powdery L-ascorbic acid to yield a homogenous mixture. Thus, 6 g of an agent for treating mitochondrial disease of the present invention was obtained as a powder passing through a #42 sieve (350 micron).

### PREPARATION EXAMPLE 2

In a ceramic mortar were placed and sufficiently ground 1 g of L-arginine, 0.2 g of powdery ascorbic acid and 1 g of powdery crude RNA of brewery yeast (RNA content: 70%) to yield a homogenous mixture. Thus, 2.2 g of an agent for treating mitochondrial disease of the present invention was obtained as a powder passing through #42 sieve (350 µ).

The above-prepared two agents for treating mitochondrial disease were subjected to a test of taste on intensity of harsh taste and sourness by subjects.

As a result, none of the agents for treating mitochondrial disease invited harsh taste and acrid feeling derived from taken L-arginine, since the weight ratio of L-arginine to L-ascorbic acid in the agents is 1/5. No sample became brown over a long period of time.

### PREPARATION EXAMPLE 3

L-arginine was mixed with and dissolved in an injection containing L-ascorbic acid but no cysteine (product of Fuso Pharmaceutical Industries, Ltd.) in a proportion of 5 parts by weight per 1 part by weight of L-ascorbic acid contained in the injection, to yield an agent for treating mitochondrial disease of the present invention in the form of a solution.

### CLINICAL EXAMPLE 1

The agent for treating mitochondrial disease of the present invention was administered to a patient with MELAS, mitochondrial disease, at a dose in terms of L-arginine of 0.5 g/kg/hr. The patient showed stroke-like episodes in the acute phase.

Most of the stroke-like episodes in the acute phase were remedied. The administration did not invite, for example, a harsh taste, acrid feeling and vomiting of the patient.

### CLINICAL EXAMPLE 2

The agent for treating mitochondrial disease of the present invention was administered to a patient with mitochondrial disease at a dose in terms of L-arginine of 0.5 g/kg/hr.

When L-arginine alone was administered, the patient underwent repetitive vomiting due to the harsh taste of L-arginine, and the effects of L-arginine did not sufficiently exhibit. The administered agent for treating mitochondrial disease of the present invention, however, did not invite vomiting but lead to improvements in the symptoms of the mitochondrial disease.

### CLINICAL EXAMPLE 3

The agent for treating mitochondrial disease of the present invention in the form of a solution was administered to a patient with MELAS in the acute phase by drip infusion. The dose of the drip infusion was controlled by periodically measuring the NOₓ level in the urine of the patient using a tes-tape so as to avoid increase in the NOₓ level.

The administration of the agent for treating mitochondrial disease of the present invention immediately resolved the stroke-like episodes specific to such patients with MELAS.

### Industrial Applicability

The present invention provides an agent that can efficaciously treat the mitochondrial disease, against which no efficacious treatment agent has been present.

The agent for treating mitochondrial disease of the present invention comprises L-arginine which works as a source of NO radicals, and the administration of the agent increases the blood L-arginine level. This can improve acute ischaemic disorders in the cerebral artery in the stroke-like episodes occurred in the acute phase of the mitochondrial disease.

The agent for treating mitochondrial disease of the present invention further comprises L-ascorbic acid in addition to L-arginine, can avoid the harsh taste and acrid feeling derived from taken L-arginine and prevent the formation of harmful substances derived from excessive NO radicals. In addition, L-ascorbic acid has effects to accelerate the treatment of the mitochondrial disease:

When further comprising ribonucleic acids, the agent for treating mitochondrial disease of the present invention accelerate the utilization of the ribonucleic acids as a nutrient for the nerve by the coadministration of arginine.

## Claims

1. An agent for treating mitochondrial disease, comprising L-arginine and 0.2 to 20 parts by weight of L-ascorbic acid per 1 part by weight of L-arginine.

2. The agent for treating mitochondrial disease according to claim 1, wherein L-ascorbic acid is contained in an amount of 0.2 to 6 parts by weight per 1 part by weight of L-arginine.

3. The agent for treating mitochondrial disease according to claim 2, wherein L-ascorbic acid is contained in an amount of 0.2 to 0.25 part by weight per 1 part by weight of L-arginine.

4. An agent for treating mitochondrial disease, comprising L-arginine, at least one selected from the group consisting of ribonucleic acids, ribonucleotides and ribonucleosides, and 0.2 to 20 parts by weight of L-ascorbic acid per 1 part by weight of L-arginine.

5. The agent for treating mitochondrial disease according to claim 4, wherein L-ascorbic acid is contained in an amount of 0.2 to 6 parts by weight per 1 part by weight of L-arginine.

6. The agent for treating mitochondrial disease according to claim 5, wherein L-ascorbic acid is contained in an amount of 0.2 to 0.25 part by weight per 1 part by weight of L-arginine.

7. An agent for treating mitochondrial disease, comprising L-arginine and 0.2 to 20 parts by weight of L-ascorbic acid per 1 part by weight of L-arginine and containing no cysteine, L-arginine and L-ascorbic acid being dissolved in a pharmaceutically acceptable solution.

8. The agent for treating mitochondrial disease according to claim 7, wherein L-ascorbic acid is contained in an amount of 0.2 to 6 parts by weight per 1 part by weight of L-arginine.

9. The agent for treating mitochondrial disease according to claim 8, wherein L-ascorbic acid is contained in an amount of 0.2 to 0.25 part by weight per 1 part by weight of L-arginine.
